(19) Europäisches Patentamt
European Patent Office
Office européen des brevets

(11) Veröffentlichungsnummer: **0 031 436**
**B1**

(12) # EUROPÄISCHE PATENTSCHRIFT

(45) Veröffentlichungstag der Patentschrift: **15.02.84**

(21) Anmeldenummer: **80107093.9**

(22) Anmeldetag: **15.11.80**

(51) Int. Cl.³: **C 07 C 51/58**, C 07 C 45/00, C 07 C 41/00, C 07 F 7/12, C 07 C 51/54, C 07 D 307/88, C 07 D 307/89, C 07 C 47/54, C 07 C 47/55, C 07 C 63/10, C 07 C 63/15

(54) **Verfahren zur Spaltung von Siloxanen.**

(30) Priorität: **13.12.79 DE 2950030**

(43) Veröffentlichungstag der Anmeldung: **08.07.81 Patentblatt 81/27**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**JOURNAL OF THE CHEMICAL SOCIETY, CHEMICAL COMMUNICATIONS, Nr. 1, 5 Januar 1977, London, T. NAKANO et al. "Convenient Synthesis of aromatic Acid Chlorides. The Reaction of Benzylidyne Chlorides with Hexamethyldisiloxane" Seiten 808 bis 809**
**Chemical Abstracts Band 45, Nr. 13, 10 Juli 1951, Columbus, Ohio, USA, BRITISH THOMSON-HOUSTON CO. LTD. "Organo-substituted halosilanes" Seite 1951, Spalte 5714a**

(73) Patentinhaber: **DYNAMIT NOBEL AKTIENGESELLSCHAFT, Patentabteilung Postfach 1209, D-5210 Troisdorf, Bez. Köln (DE)**

(72) Erfinder: **Kötzsch, Hans-Joachim, Dr., Fecampring 28, D-7888 Rheinfelden (DE)**
Erfinder: **Amort, Jürgen, Dr., Ubierstrasse 9, D-521 Troisdorf (DE)**
Erfinder: **Vahlensieck, Hans-Joachim, Dr., Im Habiken 2, D-7867 Wehr (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Spaltung von Siloxanen

Gegenstand der vorliegenden Erfindung ist ein Verfahren, bei dem man gleichzeitig Chlorsilane und aromatische Aldehyde oder Carbonsäurechloride oder Äther ohne grösseren Anfall von Nebenprodukten erhält.

Chlorsilane und Carbonsäurechloride bzw. aromatische Aldehyde sind bekannte, in vielen technischen Synthesen verwendete, Zwischenprodukte für die Herstellung von Silylierungsprodukten oder Schutzgruppen-Reagenzien sowie von Farbstoffen, Pharmazeutica und Riechstoffen.

Chlorsilane fallen in technischen Mengen bei dem als «Rochow-Synthese» bekannten Verfahren an. Nachteilig bei diesem Verfahren ist die Uneinheitlichkeit der Verfahrensprodukte, die eine fraktionierte Destillation notwendig macht, wobei ein grosser Anteil an unverwertbaren Nebenprodukten anfällt.

Aromatische Aldehyde und Carbonsäurechloride werden technisch durch Umsetzung von Chlormethylbenzolen mit Wasser oder aromatischen Carbonsäuren unter Abspaltung von Chlorwasserstoff in Gegenwart von Katalysatoren hergestellt. Bei diesen Verfahren bereitet die Durchführung der Hydrolyse verfahrenstechnische Schwierigkeiten, oder man muss die in gesonderten Verfahrensschritten hergestellten Carbonsäuren als Ausgangsprodukte verwenden, wodurch das Gesamtverfahren sehr aufwendig wird. Alle Verfahren besitzen zusätzlich noch den grossen Nachteil, dass der entstehende Chlorwasserstoff bei der Reaktion ungenutzt bleibt, aus dem Reaktionsgefäss entfernt und anschliessend einer Absorption und/oder Reinigung zugeführt werden muss. Ausserdem bewirkt der Chlorwasserstoff bei manchen der genannten Umsetzungsprodukte, z.B. dem 3-Chlorphthalid, eine verminderte Lagerstabilität; er muss deshalb aus dem Umsetzungsprodukt vollständig entfernt werden. Dieser zusätzliche Verfahrensschritt erfordert oft einen erheblichen Arbeitsaufwand.

Weiterhin sind viele Anwendungsgebiete der aus Chlorsilanen hergestellten Siloxane bzw. Silikone oder den Trialkylsilylverbindungen damit belastet, dass dabei Nebenprodukte anfallen, für deren Verwertung nur wenig Möglichkeiten bestehen. Solche nicht verwertbaren Nebenprodukte entstehen unter anderem bei der Synthese von Antibiotika (z.B. Cephalosporin-Derivaten) aufgrund des Schutzes von empfindlichen Gruppierungen durch Silylgruppen in Form von Hexamethylsiloxanen oder beim Einsatz von Siloxanen als Hydrauliköle oder Wärmeüberträger in Form des verbrauchten oder verschmutzten Öles. Oft müssen diese Nebenprodukte vernichtet werden, da z.B. die Aufarbeitung durch die an sich bekannte Spaltung von Siloxanen mit Chlorwasserstoff in Gegenwart von Schwefelsäure oder $AlCl_3$ einen hohen Aufwand erfordert und nicht optimal ist.

Es bestand deshalb die Aufgabe, diese Nebenprodukte wirtschaftlich zu verwerten und nach Möglichkeit in den gleichen Produktionszyklus zurückzuführen. Weiterhin ist es ein seit langer Zeit anstehendes Problem, die technische Herstellung von Alkylchlorsilanen zu verbessern und sie von der mit Nachteilen behafteten Rochow-Synthese unabhängig zu machen. Das dritte, zu lösende Problem war die Auffindung eines Verfahrens zur Herstellung aromatischer Carbonylverbindungen aus chlorierten Methylbenzolen, bei dem der anfallende Chlorwasserstoff direkt verwertet wird.

In Erfüllung dieser Aufgaben wurde nun das im kennzeichnenden Teil des vorliegenden Patentanspruchs beschriebene Verfahren gefunden.

Es ist zwar schon bekannt, aromatische Säurechloride aus den entsprechenden Halogenmethylbenzolen durch Umsetzung mit Hexamethyldisiloxan herzustellen (J. Chem. Soc. Chemical Communications No. 1 (1977), S. 808/809). Bei diesem Verfahren wird Eisenchlorid allein als Katalysator eingesetzt. Die Ausbeuten sind unbefriedigend. Bei dem vorliegenden Verfahren dagegen, bei dem zusätzlich noch ein Cokatalysator eingesetzt wird, erhält man nahezu quantitative Ausbeuten.

Die erfindungsgemässe Verfahrensweise ermöglicht es, Siloxane mittels Halogenmethylaromaten zu spalten, ohne dass Nebenreaktionen in nennenswerter Weise auftreten. Es entstehen dabei sowohl aus den Siloxanen als auch aus den Halogenmethylaromaten technisch verwertbare, wertvolle Endprodukte, die in hoher Reinheit anfallen. Eine Verunreinigung durch Chlorwasserstoff tritt nicht oder nur in untergeordnetem Masse auf.

Die erfindungsgemässe Arbeitsweise lässt sich praktisch mit allen Siloxanen durchführen, so dass man Halogensilane mit wählbaren Substituenten erhalten kann, je nachdem, welche Siloxane als Ausgangsmaterial eingesetzt werden.

Die Halogenmethylbenzole, die erfindungsgemäss einsetzbar sind, umfassen Mono-, Di- und Trihalogenmethylbenzole, unabhängig davon, wieviele und in welcher Stellung zueinander die halogenierten Methylgruppen am Benzolring stehen. Bei den Monohalogenmethylbenzolen können jedoch, je nach Ausgangssubstanz, geringere Ausbeuten an gewünschtem Endprodukt eintreten. Das Prinzip der neu aufgefundenen Reaktion ist jedoch auf diese Verbindungen ebenfalls anwendbar.

Die erfindungsgemässe Reaktion lässt sich durch folgende Gleichungen zusammenfassen.

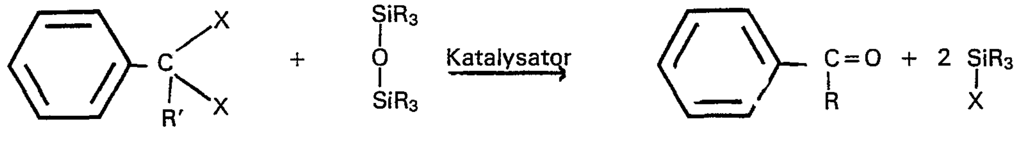

(1)

und

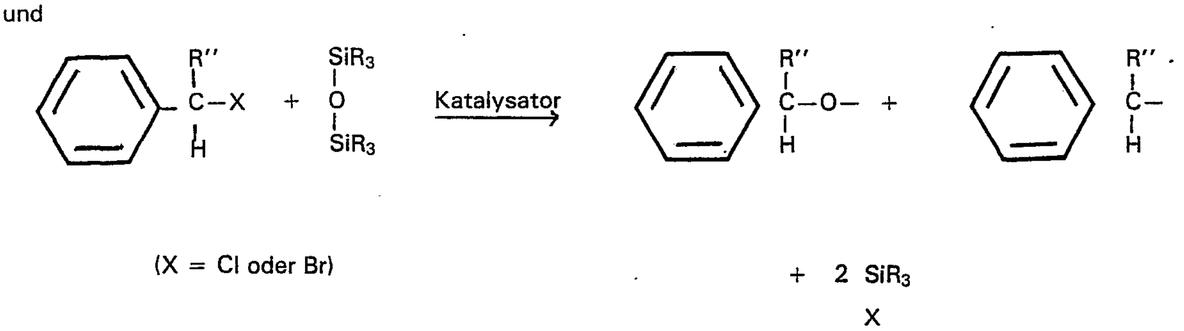

(2)

(X = Cl oder Br)

In der Gleichung (1) steht R' für Wasserstoff, Halogen oder einen aromatischen Rest, bevorzugt Phenylrest. In der Gleichung (2) kann R'' für die in Gleichung (1) genannten Reste und für ein Sauerstoffatom stehen, das über eine Carbonylgruppe mit dem Benzolring verbunden ist. Die gemäss Gleichung (2) entstehenden aromatischen Reste reagieren bevorzugt intermolekular miteinander, z.B. unter Bildung von Diphthalidyläther oder Dibenzyläther.

Bei der Reaktion mit Monohalogenmethylbenzolen können sich intermediär neben den Halogenalkylsilanen auch Benzyltrialkylsilyläther der allgemeinen Formel

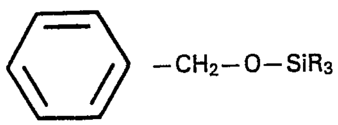

bilden, entsprechend der Gleichung (3).

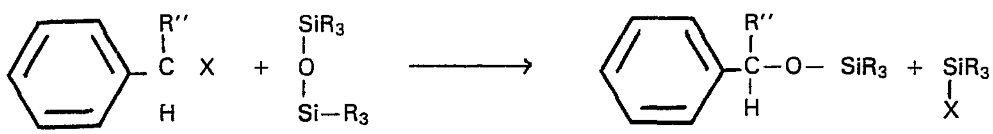

(3)

(X = Cl oder Br) (R = Halogen, Alkyl, Alkenyl oder $-OSiR_3$)

In manchen Fällen lässt sich die Bildung dieses Äthers direkt (z.B. gaschromatographisch) nachweisen; unter den Reaktionsbedingungen reagiert er jedoch leicht weiter unter Bildung der oben genannten Endprodukte.

Es ist für den Ablauf der erfindungsgemässen Reaktion unerheblich, ob am Benzolkern weitere Substituenten, wie z.B. Halogen oder unsubstituierte Alkylreste, sich befinden.

Die allgemeinen Gleichungen (1) und (2) zeigen, dass pro Äquivalent abspaltbarem Halogen am Halogenmethylbenzol 0,5 Mol der Gruppierung $R_3Si$-O-$SiR_3$ eingesetzt werden. Wenn man gemäss Gleichung (3) die als Zwischenprodukte entstehenden Silyläther isolieren will, muss man in diesem Fall pro Monohalogenmethylbenzol ein Äquivalent der Gruppierung $R_3Si$-O-$SiR_3$ einsetzen.

Bei der erfindungsgemässen Umsetzung entstehen je nach dem Halogenierungsgrad der Methylgruppe aromatische Carbonsäurechloride, aromatische Aldehyde oder Ketone oder Äther.

Als Katalysator für das erfindungsgemässe Verfahren eignen sich eine Grosszahl von Metallen, elementar oder in gebundener Form. Prinzipiell zeigen alle Metalle der Nebengruppenelemente oder der 5. Hauptgruppe des Periodischen Systems der Elemente eine mehr oder minder ausgeprägte katalytische Wirkung. Besonders wirksame Metallkatalysatoren im Sinne des erfindungsgemässen Verfahrens sind Eisen und Nickel, Mangan, Molybdän, Vanadium, Arsen, Antimon, Wismut, Thallium, Zink und Cadmium. Sie wirken sowohl bei Zugabe in elementarer Form, vorzugsweise als Pulver, als auch in Form ihrer Verbindungen (z.B. als Oxide) oder ihrer Salze, beispielsweise als Acetylacetonate, Sulfate, Sulfide, Chloride, Acetate, Silikate, Phosphate aber auch in Alkoholatform. Bevorzugt eingesetzt werden die Chloride. Diese Katalysatoren können in Substanz oder, sofern sie in einem der Reaktionsteilnehmer oder dem Cokatalysator löslich sind, auch in gelöster Form eingesetzt werden. Bei Verwendung dieser Katalysatoren ist als weitere Verfahrensbedingung für die Ausübung des erfindungsgemässen Verfahrens die Anwesenheit eines Protonen-Donators als Cokatalysator zum Starten der Reaktion erforderlich. Es ost dann von Vorteil, den Katalysator damit zu kombinieren.

Als Katalysatoren eignen sich weiterhin Sauerstoffsäuren des Schwefels. Darunter fallen sowohl anorganische als auch organische Sauerstoffsäuren, die gegebenenfalls auch substituiert sein können. Als Beispiele seien genannt: Schwefelsäure, Thioschwefelsäure, Chlorsulfonsäure, p-Toluolsulfonsäure.

Die Aufgabe des Cokatalysators für das erfindungsgemässe Verfahren erfüllt jede Substanz, aus der ein Wasserstoffkation abgespalten werden kann: z.B. Wasser, jede Protonsäure, Carbonsäuren, Alkoholate etc. Sofern die Katalysatoren neben ihrer katalytischen Aktivität nicht schon selbst auch Cokatalysatoreigenschaft besitzen, z.B. die Säuren des Schwefels, oder einen Kristallwassergehalt in Salzen z.B. $FeCl_3 \cdot 6\ H_2O$, ist es daher von Vorteil, die löslichen Katalysatorverbindungen vor Gebrauch vorzugsweise in Wasser oder wässrigen Säuren zu lösen; unlösliche Verbindungen sind jedoch in Substanz einzusetzen und der Cokatalysator ist dann gesondert zuzusetzen, um die Reaktion damit zu starten.

Der Katalysator, sowie gegebenenfalls der Cokatalysator, wird zu Beginn der Umsetzung dem Reaktionsgemisch zugeführt. Es ist nicht notwendig, dass der Katalysator im Reaktionsmedium gelöst vorliegt. Es sind bereits Mengen von $10^{-4}$ Gew.-%, bezogen auf das Reaktionsgemisch, wirksam. Auch noch geringere Mengen des Katalysators sind in manchen Fällen ebenfalls wirksam.

Bevorzugt wird der Katalysator in Mengen zwischen $10^{-4}$ und 1 Gew.-%, bezogen auf das Reaktionsgemisch, eingesetzt. Prinzipiell ist es auch möglich, grössere Mengen einzusetzen; doch bringt ein solcher Zusatz im allgemeinen keine oder nur geringfügige Verbesserungen.

Die Menge des Cokatalysators liegt in der gleichen Grössenordnung wie diejenige des Katalysators.

Die erfindungsgemässe Reaktion läuft im Temperaturbereich zwischen 90 und 220 °C ab. Die bevorzugten Temperaturen liegen zwischen 135 und 175 °C. Die Reaktionstemperatur soll möglichst oberhalb des Siedepunkts des entstehenden Chlorsilans liegen, damit dieses im Verlauf der Reaktion kontinuierlich abdestilliert werden kann.

Die Reaktion läuft sehr schnell ab. Es ist deshalb empfehlenswert, einen der Reaktionspartner, vorzugsweise das Halogenmethylbenzol, zusammen mit dem Katalysator und gegebenenfalls dem Cokatalysator, vorzulegen und den zweiten Reaktionspartner dem auf die gewünschte Reaktionstemperatur erhitzten Reaktionspartner hinzuzufügen. Es ist dabei von Vorteil, auch den zweiten Reaktionspartner auf die Reaktionstemperatur aufzuheizen.

Das bei der Reaktion entstehende Halogenalkylsilan wird zweckmässigerweise im Zuge seiner Entstehung abdestilliert. Der zweite Reaktionspartner wird dann vorzugsweise mit der Geschwindigkeit hinzugefügt, mit der das Halogenalkylsilan abdestilliert wird. Die Zuführungsgeschwindigkeit der zweiten Reaktionskomponente ist also abhängig von der Destillationskapazität der Kolonne, über die das Halogensilan abdestilliert wird. Dabei ist jedoch darauf zu achten, dass die Reaktion nicht unterbrochen wird, weil sonst nochmals frischer Katalysator und/oder Cokatalysator hinzugefügt werden muss. Sobald die Reaktion angesprungen ist, sollte also im Reaktionsmedium immer eine geringe Menge des zweiten Reaktionspartners anwesend sein.

Nach Beendigung der Zuführung des zweiten Reaktionspartners ist bei der geschilderten Arbeitsweise auch unmittelbar anschliessend die Destillation des Halogensilans beendet. Dieses fällt in Ausbeuten über 90% und in hoher Reinheit an; es kann direkt einer Weiterverarbeitung zugeführt werden.

Die Isolierung der aromatischen Carbonylverbindungen bzw. der aromatischen Äther erfolgt anschliessend auf an sich bekannte Weise entweder durch Destillation, gegebenenfalls im Vakuum, oder durch Auskristallisation. Im letzteren Falle verwendet man als Reaktor einen solchen mit Rührwerk für Feststoffe, beispielsweise mit randgängigem Ankerrührer, und hält während der Umsetzung das Reaktionsgemisch in steter Bewegung.

Für die Reaktion wird im allgemeinen ein Reaktor eingesetzt, der gleichzeitig Blase einer Destillationskolonne ist. Der Reaktor enthält weiterhin ein in die flüssige Phase der vorgelegten Ausgangsverbindung reichendes Einleitungsrohr zur Einleitung der zweiten, flüssigen Reaktionskomponente, die gegebenenfalls auch in Gasform (z.B. vorgewärmt oder verdampft) über dieses Einleitungsrohr in die erste Reaktionskomponente eingeführt werden kann.

Die erfindungsgemäss erhaltenen aromatischen Carbonylverbindungen bzw. Äther fallen ebenfalls in über 90%iger Ausbeute und in einer Reinheit an, die ihre sofortige Weiterverwendung ohne zusätzliche Reinigungsoperationen ermöglicht. Wenn dieses Verfahrensprodukt kristallin ist, ist für den Fall, dass ein besonders reines Produkt gewünscht wird, lediglich eine Umkristallisation notwendig.

Die als Ausgangsprodukte einsetzbaren Chlormethylbenzole umfassen Verbindungen der allgemeinen Formel

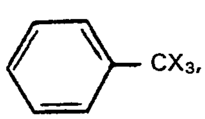 , 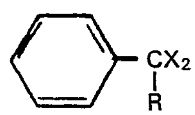 und 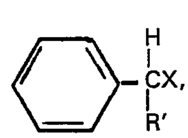 ,

in denen R für Wasserstoff, einen Arylrest, bevorzugt den Phenylrest, oder einen Alkyl- oder Alkenylrest mit bevorzugt 1-4 C-Atomen steht und R' gleich R oder ein Sauerstoffatom ist, das mit dem Benzolkern über eine Carbonylgruppe verbunden ist. X steht für Chlor oder Brom. Der Benzolkern kann weiterhin durch Halogen oder unsubstituierte Alkylreste ein- oder mehrmals substituiert sein. Auch Verbindungen, bei denen die Halogenmethylgruppe mehrmals am Benzolkern steht, können erfindungsgemäss eingesetzt werden.

Als Beispiele für Verbindungen, die unter diese Formeln fallen, seien genannt: Benzalchlorid, p-Fluorbenzalchlorid, p-Chlorbenzalchlorid, 2,4-Dichlorbenzalchlorid, p-Brombenzalchlorid, Benzotrichlorid, 3-Chlorbenzotrichlorid, 4-Chlorbenzotrichlorid, 2,4-Dichlorbenzotrichlorid, 2,4,6-Trichlorbenzotrichlorid, p-Brombenzotrichlorid, 3,5-Dibrombenzotrichlorid, 2,4-Dibrombenzotrichlorid, p-Jodbenzotrichlorid, Fluoridchlormethylbenzol, $\alpha,\alpha'$-Pentachlor-o-xylol, 1,3[Bis-trichlormethyl]benzol, 1,4-[Bis-trichlormethyl]benzol, 1,4[Bis-trichlormethyl],

2,3,5,6-tetrachlorbenzol, 2,3,5,6-Tetrabrom-α,α'-hexachlor-p-xylol und Diphenyldichlormethan.

Erfindungsgemäss werden aus solchen aromatischen Chlormethylverbindungen Aldehyde oder Carbonsäurechloride oder deren Derivate hergestellt, beispielsweise Benzaldehyd, p-Fluorbenzaldehyd, p-Chlorbenzaldehyd, 2,4-Dichlorbenzaldehyd, p-Brombenzaldehyd, p-Jodbenzaldehyd, Benzoylchlorid, 3-Chlorbenzoylchlorid, 4-Chlorbenzoylchlorid, 2,4-Dichlorbenzoylchlorid, 2,4,6-Trichlorbenzoylchlorid, p-Brombenzoylchlorid, 3,5-Dibrombenzoylchlorid, 2,4-Dibrombenzoylchlorid, p-Jodbenzoylchlorid, Benzoylfluorid, 3-Chlorphthalid, Di-phthalidyläther, Isophthalsäuredichlorid, Terephthalsäuredichlorid, Tetrachlorterephthalsäuredichlorid, Tetrabromterephthalsäuredichlorid usw.

Die weiterhin als Ausgangsstoffe einsetzbaren Siloxane umfassen alle flüssigen Siloxane, die die Gruppierung $\geqslant Si-O-Si \leqslant$ besitzen, wobei die freien Valenzen des Si-Atoms durch Halogen, Alkyl, Alkenyl oder eine weitere $\geqslant Si-O-Si \leqslant$-Gruppierung abgesättigt sein kann. Unter diesen Verbindungen sollen beispielsweise verstanden werden:

Disiloxane der allgemeinen Formel $(R''_{3-a}Cl_aSi)_2O$ (a = 0-3), R'' = Alkyl oder Alkenyl mit 1-4 C-Atomen; Cyclopolysiloxane der allgemeinen Formel $(R''_{2-b}Cl_bSiO)_c$ (b = 0-2; C = 3-12 für monocyclische Struktur) sowie die korrespondierenden Ringe polycyclischer, spirocyclischer und sphärocyclischer Struktur;

kettenpolymere Dialkyl- bzw. Dialkenylpolysiloxane der allgemeinen Formel $R''_3SiO(R''_2SiO)_dSiR''$ und verzweigte Siloxane und/oder Polysiloxane z.B. der allgemeinen Formel $R''Si[(OSiR''_2)_dOSiR_3]_3$ (d = Polymerisationsgrad von 1 bis zu den höchsten Werten, bei denen das Polymere noch flüssig dosiert werden kann, z.B. 5000).

Einzelne Verbindungen, die unter diese Definition fallen, sind z.B. Hexachlordisiloxan, Tetramethyldichlordisiloxan, Hexamethyldisiloxan, Hexaäthyldisiloxan, Hexamethylcyclotrisiloxan, Octamethylcyclotetrasiloxan, Decamethylcyclopentasiloxan, Divinyltetrachlordisiloxan, Diisobutyltetrachlordisiloxan, lineare Polydimethylsiloxane niedriger bis hoher Polymerisationsgrade und Viskositäten, Polyvinylmethylsiloxane, Methyl-tris-trimethylsiloxisilan, Tetrakis-trimethylsiloxisilan, verzweigte Polymethylsiloxane, Siliconöle, insbesondere Siloxan- bzw. Silicon-Abfälle wie z.B. verbrauchte Wärmeträgeröle und Hydrauliköle etc.

Durch das erfindungsgemässe Verfahren entstehen daraus Chlorsilane bzw. Chlororganosilane wie z.B. Tetrachlorsilan, Methyltrichlorsilan, Vinyltrichlorsilan, Propyltrichlorsilan, Isobutyltrichlorsilan, Dimethyldichlorsilan, Vinylmethyldichlorsilan, Trimethylchlorsilan usw. je nach eingesetztem Siloxan. Verzweigte Siloxane liefern gegebenenfalls Gemische, die nach den dafür bekannten destillativen Methoden zu trennen sind.

*Beispiel 1*

Herstellung von Benzoylchlorid und Tetrachlorsilan aus Benzotrichlorid und Hexachlordisiloxan in Gegenwart von Zink (II) chlorid ($ZnCl_2$).

In einem mittels Ölbad beheiztem 2 l Vierhalskolben, ausgerüstet mit Rührer, Innenthermometer, eintauchendem Gaseinleitungsrohr und aufgesetzter Kolonne (6 Böden, ∅ 50 mm, Füllhöhe 250 mm V4A-Streckmetall für Vakuumdestillation) mit automatischem Kolonnenkopf werden 1955 g (10 Mol) Benzotrichlorid (Trichlormethylbenzol) vorgelegt und auf 148°C erwärmt. Dann wird die Reaktion gestartet durch Zugabe von 1 ml 50%iger $ZnCl_2$-Lösung in Wasser bei gleichzeitigem Beginn der Zuführung von gasförmigem Hexachlordisiloxan über das Gaseinleitungsrohr, das zu diesem Zweck über einen bei 160°C arbeitenden Verdampfer mit einem in ca. 900 mm Höhe angeordneten Vorratsgefäss für Hexachlordisiloxan verbunden ist. Das Hexachlordisiloxan wird in einer Menge von 2840 g (9,97 Mol) mit einer gleichbleibenden Geschwindigkeit von ca. 350 g pro Stunde zugeführt.

Unmittelbar nach dem Start der Reaktion setzt beim Rücklaufverhältnis 1 die Destillation von reinem $SiCl_4$ (Kp. 57,6°C) ein und endet unmittelbar nach Beendigung der Siloxanzuführung.

Anschliessend wird die Vorlage gewechselt und im Vakuum Benzoylchlorid (Kp. 92°C) bei 40 mbar (F. −1°C) ausdestilliert. Die Ausbeute an Benzoylchlorid beträgt 1348 g (96,3%) und an Tetrachlorsilan 3305 g (98,4%).

*Beispiel 2*

Herstellung von Terephthalsäuredichlorid und Trimethylchlorsilan aus α,α'-Hexachlor-p-xylol und Hexamethyldisiloxan in Gegenwart von $MoO_3$.

In einer Laboranlage analog Beispiel 1, jedoch mit 10 l Kolben, werden 12510 g (40 Mol) α,α'-Hexachlor-p-xylol auf 159°C erwärmt. Dann werden 0,5 g $MoO_3$ eingerührt und die Reaktion wird durch Zugabe von wenig gasförmigem Chlorwasserstoff in die Schmelze bei gleichzeitigem Beginn der Zuführung von gasförmigem Hexamethyldisiloxan über das Gaseinleitungsrohr analog Beispiel 1 gestartet. Das Hexamethyldisiloxan wird in einer Menge von 12974 g (79,9 Mol) mit einer gleichbleibenden Geschwindigkeit von ca. 1200 g pro Stunde zugeführt.

Unmittelbar nach dem Start der Reaktion setzt beim Rücklaufverhältnis 1 die Destillation von reinem Trimethylchlorsilan (Kp. 57,7°C) ein und endet unmittelbar nach dem Ende der Siloxanzuführung.

Anschliessend wird die Kühlertemperatur des Kolonnenkopfes mittels Thermostat auf 80°C eingestellt, die Vorlage gewechselt und im Vakuum Terephthalylchlorid (Kp. 119°C bei 18 mbar; F. 78°C) ausdestilliert.

Die Ausbeute an Terephthalylchlorid beträgt 7922 g (97,5%) und an Trimethylchlorsilan 17,26 kg (99,5%).

*Beispiel 3*

Herstellung von 3-Chlorphthalid und Trimethylchlorsilan aus α,α'-Pentachlor-o-xylol und Hexamethyldisiloxan in Gegenwart von $FeCl_3 \cdot 6H_2O$.

Analog Beispiel 1 und 2 werden 11136 g (40 Mol) α,α'-Pentachlor-o-xylol ([1-Trichlormethyl-2-dichlormethyl]benzol) auf 141°C erwärmt. Dann wird die Reaktion gestartet durch Zugabe von 1 ml 50%ig wässriger $FeCl_3 \cdot 6H_2O$-Lösung bei gleichzeitigem

Beginn der Zuführung von gasförmigem Hexamethyldisiloxan.

Das Hexamethyldisiloxan wird in einer Menge von 12970 g (79,9 Mol) mit einer gleichbleibenden Geschwindigkeit von ca. 1200 g pro Stunde zugeführt. Nach jeweils 30 Minuten werden je 0,2 ml 50%ig wässriger $FeCl_3 \cdot 6H_2O$-Lösung zum laufenden Ansatz hinzugefügt.

Unmittelbar nach dem Start der Reaktion setzt beim Rücklaufverhältnis 1 die Destillation von reinem Trimethylchlorsilan (Kp. 57,7°C) ein und endet unmittelbar nach dem Ende der Siloxanzuführung.

Anschliessend wird die Kühlertemperatur des Kolonnenkopfes mittels Thermostat auf 80°C eingestellt, die Vorlage gewechselt und im Vakuum 3-Chlorphthalid (Kp. 102°C bei 2 mbar; F. 60-61°C) ausdestilliert.

Die Ausbeute an 3-Chlorphthalid beträgt 6530 g (96,9%) und an Trimethylchlorsilan 17,22 kg (99,3%).

Die Herstellung erfolgt gemäss der Gleichung:

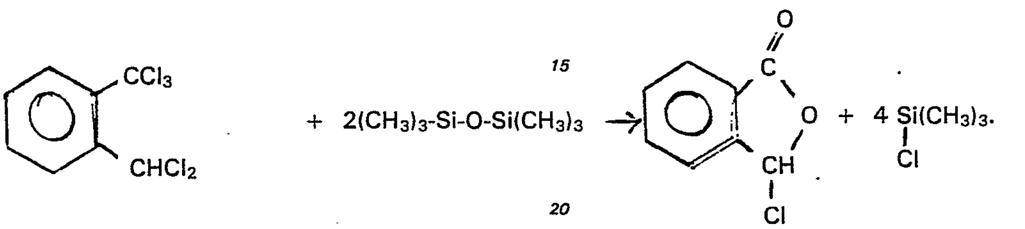

*Beispiel 4*

Herstellung von Diphthalidyläther und Trimethylchlorsilan aus $\alpha,\alpha'$-Pentachlor-o-xylol und Hexamethyldisiloxan in Gegenwart von $FeCl_3 \cdot 6H_2O$.

In einem über Doppelmantel mittels Thermostat beheizten Laborrührwerk mit 4 l Inhalt für zähflüssige und kristalline Stoffe, das mit einem randgängigen Ankerrührer (83 UpM) und Strömungsbrechern, innenliegendem Temperaturfühler, eintauchendem Gaseinleitungsrohr und aufgesetzter Kolonne (ca. 8 Böden, ∅ 50 mm, Füllhöhe 250 mm, Füllkörper 6 mm Porzellansättel) mit automatischem Kolonnenkopf ausgerüstet ist, werden 3340 g (12 Mol) $\alpha,\alpha'$-Pentachlor-o-xylol auf 140°C erwärmt. Dann wird die Reaktion gestartet durch Zugabe von 1 ml 50%ig wässriger $FeCl_3 \cdot 6H_2O$-Lösung bei gleichzeitigem Beginn der Zuführung von gasförmigem Hexamethyldisiloxan (analog Beispiel 1). Das Hexamethyldisiloxan wird in einer Menge von 4872 g (30 Mol) mit einer gleichbleibenden Geschwindigkeit von ca. 900 g pro Stunde zugeführt.

Unmittelbar nach dem Start der Reaktion setzt beim Rücklaufverhältnis 1 die Destillation von reinem Trimethylchlorsilan (Kp. 57,7°C) ein und endet unmittelbar nach dem Ende der Siloxanzuführung. Das flüssige Reaktionsgemisch beginnt nach dem Zusatz von ca. 4 kg Hexamethyldisiloxan Kristalle auszuscheiden und verwandelt sich während der Zugabe des restlichen Siloxans in ein trockenes Kristallpulver bestehend aus reinem Diphthalidyläther, der nach kurzem Evakuieren und Fluten mit Stickstoff einen Schmelzpunkt von 220 bis 222°C besitzt.

Die Ausbeute beträgt 1646 g (97%) Diphthalidyläther und 6420 g (98,5%) Trimethylchlorsilan.

Die Herstellung erfolgt gemäss der Gleichung:

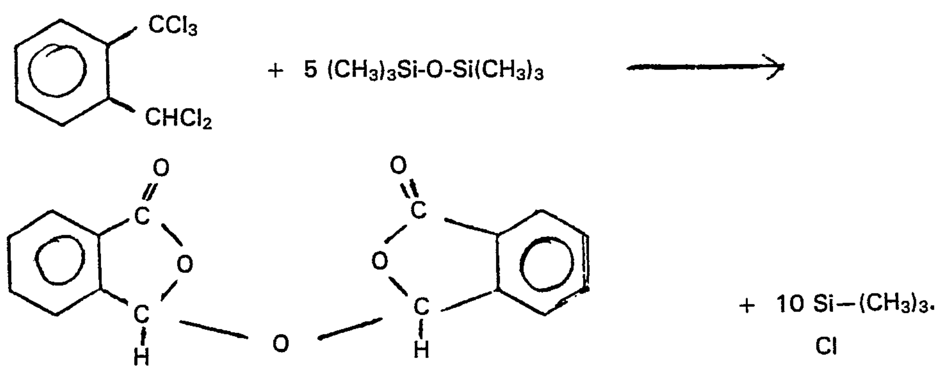

*Beispiel 5*

Herstellung von p-Chlorbenzaldehyd und Trimethylchlorsilan aus p-Chlorbenzalchlorid und Hexamethyldisiloxan in Gegenwart von $TiCl_3$.

Analog Beispiel 1 werden 1955 g (10 Mol) p-Chlorbenzalchlorid auf 166°C erwärmt.

Dann wird die Reaktion gestartet mit 1 ml 50%ig wässriger $TiCl_3$-Lösung und weiter verfahren analog Beispiel 1, jedoch mit 1640 g (10 Mol) Hexamethyldisiloxan als zweiter Reaktionskomponente, die mit der Geschwindigkeit von ca. 230 g pro Stunde zugeführt wird.

Während der Reaktion destillieren 2130 g Trimethylchlorsilan ab (ca. 98% Ausbeute).

Nach beendeter Reaktion wird die Kühlertemperatur des Kolonnenkopfes mittels Thermostat auf 50°C eingestellt und im Vakuum der p-Chlorbenzaldehyd (F. 47-48°C) ausdestilliert (Kp. 114°C bei 60 mbar; 1360 g = 96,7%).

*Beispiel 6*

Herstellung von 2,5-Dichlorbenzaldehyd und Trimethylchlorsilan aus 2,5-Dichlorbenzalchlorid und Hexamethyldisiloxan in Gegenwart von $H_2SO_4$.

Analog Beispiel 5 werden 2300 g 2,5-Dichlorbenzalchlorid bei 170°C mit 0,5 ml konz. $H_2SO_4$ als Starter und Katalysator umgesetzt.

Es werden 2126 g (ca. 98%) Trimethylchlorsilan und 1684 g (ca. 96,2%) 2,5-Dichlorbenzaldehyd (Kp. 129°C bei 20 mbar und 60°C Kühlertemperatur wegen F. 56-58°C) erhalten.

*Beispiel 7*

Herstellung von Tetrachlorterphthalsäutredichlorid und einem Gemisch bestehend aus Trimethylchlorsilan und Methyltrichlorsilan aus Perchlor-p-xylol (1,4-[Bis-Trichlor-1-methyl]2,3,5,6-Tetrachlorbenzol) und Methyl-tris-trimethylsiloxisilan in Gegenwart von $SbCl_5$.

Analog Beispiel 4 werden 4055 g (9 Mol) Perchlor-p-xylol bei 158°C mit 0,5 ml $SbCl_5$ verrührt und die Reaktion mit 1 ml Wasser gestartet bei gleichzeitigem Beginn der Zuführung von 1860 g (6 Mol) $MeSi[OSi(CH_3)_3]_3$, das auf 160°C vorgeheizt wird, in flüssiger Form über das Tauchrohr mit gleichbleibender Geschwindigkeit von ca. 380 g pro Stunde. Während der Reaktionszeit destilliert ein Gemisch von Trimethylchlorsilan und Methyltrichlorsilan bei einer Kopftemperatur von ca. 59°C über;
insgesamt ca. 2800 g (ca. 98%) mit einem gaschromatographisch ermittelten Verhältnis der beiden Produkte von ca. 7 : 3.

Aus dem Reaktionsgefäss werden nach dem Kaltrühren, Evakuieren auf 20 mbar nach dem Kristallisieren bei ca. 140°C und Fluten mit $N_2$ 3040 g (ca. 99% Ausbeute) Tetrachlorphthalsäuredichlorid (F. 142-143°C) erhalten.

*Beispiel 8*

Herstellung von p-Brombenzoylchlorid und Dimethyldichlorsilan aus p-Brombenzotrichlorid und Hexamethylcyclotrisiloxan in Gegenwart von $BiCl_3$.

Analog Beispiel 1 werden 1646 g (6 Mol) p-Brombenzotrichlorid auf 154°C erwärmt, mit 0,5 $BiCl_3$ verrührt und die Reaktion mit 1 ml Wasser gestartet bei gleichzeitigem Beginn der Zuführung von 445 g (2 Mol) Hexamethylcyclotrisiloxan (gasförmig durch Vorheizen auf Reaktionstemperatur).

Während der Reaktionszeit (ca. 4 Stunden) destillieren beim Rücklaufverhältnis 1 ca. 770 g Dimethyldichlorsilan (Kp. 70°C) in nahezu quantitativer Ausbeute über Kopf. Danach werden bei einer Kühlertemperatur von ca. 42°C im Vakuum 1245 g (ca. 95% Ausbeute) p-Brombenzoylchlorid (F. 39-40°C) ausdestilliert.

*Beispiele 9 bis 12*

Herstellung von Isophthalsäuredichlorid und Trimethylchlorsilan aus $\alpha,\alpha'$-Hexachlor-m-xylol und Hexamethyldisiloxan in Gegenwart der Katalysatoren $CdCl_2 \cdot H_2O$, $NiCl_2 \cdot 6H_2O$, $MnCl_2 \cdot 4H_2O$ und Paratoluolsulfonsäure.

Analog Beispiel 1 werden vier Versuche mit je 1565 g (5 Mol) $\alpha,\alpha'$-Hexachlor-m-xylol bei ca. 150°C mit je 1620 g (10 Mol) Hexamethyldisiloxan durchgeführt. Als Katalysatoren und Starter wird je Versuch 1 ml 50%iger wässriger Lösung von $CdCl_2 \cdot H_2O$, $NiCl_2 \cdot 6H_2O$, $MnCl_2 \cdot 4H_2O$ oder Paratoluolsulfonsäure verwendet. Während der Reaktion wird Trimethylchlorsilan (Kp. 57,7°C) abdestilliert. Danach wird im Vakuum bei der Kühlertemperatur von 44°C das erhaltene Isophthalsäuredichlorid (Kp. 97°C bei 3 mbar; F. 40-41°C) destilliert.

Die Versuchsverbindungen und Ergebnissen sind in nachstehender Tabelle zusammengefasst:

| Beispiel Nr. | Katalysator | Isophthalsäuredichlorid Auswaage | %Ausbeute | Trimethylchlorsilan Auswaage | %Ausbeute |
|---|---|---|---|---|---|
| 9 | $CdCl_2 \cdot H_2O$ | 977 g | 96 % | 2154 g | 99 % |
| 10 | $NiCl_2 \cdot 6H_2O$ | 986 g | 97 % | 2148 g | 99 % |
| 11 | $MnCl_2 \cdot 4H_2O$ | 980 g | 96,5% | 2142 g | 98,5% |
| 12 | $Ch_3$- 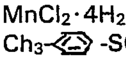 -$SO_3H$ | 975 g | 96 % | 2132 g | 98 % |

**Patentansprüche**

1. Verfahren zur Spaltung von Siloxanen und gleichzeitigen Herstellung von Halogensilanen und ein- oder zweikernigen aromatischen Carbonylverbindungen mit mindestens einer Carbonylgruppe oder ein- oder zweikernigen aromatischen Ethern, bei dem Mono- oder di(halogenmethyl)benzole, deren Methylgruppe ggf. durch einen Phenylrest substituiert ist und bei denen der Benzolkern durch Halogen oder unsubstituierte Alkylreste ein- oder mehrmals substituiert sein kann, mit Siloxanen der allgemeinen Formel

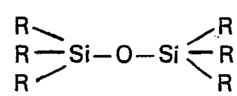

in der die Reste R gleiche oder verschiedene Reste aus der Gruppe Halogen, Alkyl, Alkenyl oder $O-SiR_3$ sind, in der R die gleiche Bedeutung wie oben hat und ggf. mindestens zwei dieser Reste gemeinsamer Bestandteil einer Ringverbindung sind, in Gegenwart von Katalysatoren umgesetzt werden, dadurch gekennzeichnet, dass man die Umsetzung bei erhöhter Temperatur in Gegenwart katalytischer Mengen von Metallen oder Metallverbindungen der Nebengruppenelemente oder der 5. Hauptgruppe des periodischen Systems der Elemente zusammen mit einem Protonen-Donator oder in Gegenwart von katalytischen Mengen von Sauerstoffsäuren des Schwefels durchführt.

2. Verfahren gemäss Anspruch 1, dadurch gekennzeichnet, dass die Halogenmethylgruppe des Mono- oder dihalogenmethylbenzols eine Di- oder Trichlormethylgruppe ist.

3. Verfahren gemäss Ansprüchen 1 oder 2, dadurch gekennzeichnet, dass die Reaktion bei Temperaturen zwischen 90 und 220°C durchgeführt wird.

4. Verfahren gemäss Anspruch 1 bis 3, dadurch gekennzeichnet, dass man das bei der Reaktion entstehende Halogensilan im Verlauf der Umsetzung kontinuierlich abdestilliert.

5. Verfahren gemäss einem der Ansprüche 1 bis 4, dadurch gekennzeichnet, dass man die aromatische Halogenmethylverbindung zusammen mit dem Katalysator vorlegt, auf Reaktionstemperatur erhitzt und das Siloxan mit einer solchen Geschwindigkeit hinzufügt, die etwa derjenigen Geschwindigkeit entspricht, mit der das entsprechende Halogensilan aus dem Reaktionsgemisch entfernt wird.

**Revendications**

1. Procédé en vue de dissocier des siloxanes et de préparer simultanément des halogénosilanes, ainsi que des composés carbonylés aromatiques à un ou deux noyaux comportant au moins un groupe carbonyle ou des éthers aromatiques à un ou deux noyaux, procédé dans lequel on fait réagir des mono- ou des di(halogénométhyl)benzènes dont le groupe méthyle est éventuellement substitué par un groupe phényle et dans lesquels le noyau benzène peut être substitué une ou plusieurs fois par un atome d'halogène ou par des groupes alkyle non substitués, avec des siloxanes de formule générale:

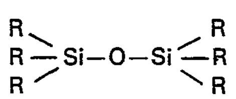

dans laquelle les radicaux R sont identiques ou différents et sont choisis parmi le groupe comprenant les atomes d'halogènes, les groupes alkyle, les groupes alcényle ou le groupe O-$SiR_3$ où R a la même signification que celle indiquée ci-dessus et éventuellement deux de ces radicaux sont un constituant commun d'un composé cyclique, en présence de catalyseurs, caractérisé en ce qu'on effectue la réaction à température élevée en présence de quantités catalytiques de métaux ou de composés métalliques des éléments des sous-groupes ou du cinquième groupe principal du Système Périodique des Eléments, avec un donneur de protons ou en présence de quantités catalytiques d'acides oxygénés du soufre.

2. Procédé suivant la revendication 1, caractérisé en ce que le groupe halogénométhyle du mono- ou du dihalogénométhylbenzène est un groupe di- ou trichlorométhyle.

3. Procédé suivant la revendication 1 ou 2, caractérisé en ce qu'on effectue la réaction à des températures comprises entre 90 et 220°C.

4. Procédé suivant les revendications 1 à 3, caractérisé en ce que, au cours de la réaction, on sépare continuellement l'halogénosilane formé lors de la réaction, par destillation.

5. Procédé suivant une des revendications 1 à 4, caractérisé en ce qu'on dépose préalablement le composé halogénomethyle aromatique avec le catalyseur, on chauffe à la température réactionnelle et on ajoute le siloxane à une vitesse calculée de telle sorte qu'elle corresponde à peu près à celle avec laquelle l'halogénosilane correspondant est éliminé du mélange réactionnel.

**Claims**

1. Process for the cleavage of siloxanes and simultaneous production of halosilanes and mono- or binuclear aromatic carbonyl compounds with at least one carbonyl group or mono or binuclear aromatic ethers, in which mono- or di(halomethyl)benzenes whose methyl group is optionally substituted by a phenyl group and in which the benzene nucleus can be substituted by halogen or unsubstituted alkyl groups one or more times, are reacted in the presence of catalysts with siloxanes of the general formula

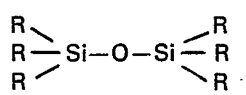

in which the residues R are like or different residues from the group of halogen, alkyl, alkenyl or O-$SiR_3$, in which R has the same meaning as above and optionally at least two of these residues are common component of a ring compound, characterised in that the reaction is carried out at elevated temperature in the presence of catalytic amounts of metals or metal compounds of sub-group elements or of the fifth main group of the Periodic System of the Elements together with a proton donor or in the presence of catalytic amounts of oxy acids of sulphur.

2. Process according to claim 1, characterised in that the halomethyl group of the mono or dihalomethyl benzene is a di or trichloromethyl group.

3. Process according to claim 1 or 2, characterised in that the reaction is carried out at temperatures between 90 and 220°C.

4. Process according to claim 1 to 3, characterised in that the halosilane existing in the reaction is continuously distilled off in the course of the reaction.

5. Process according to one of claims 1 to 4, characterised in that the aromatic halomethyl compound is supplied together with the catalyst, heated to reaction temperature and the siloxane is added at such a rate which corresponds to about that rate with which the corresponding halosilane is removed from the reaction mixture.